# EUROPÄISCHE PATENTSCHRIFT

(11) **EP 1 711 190 B1**
(45) Veröffentlichungstag und Bekanntmachung des Hinweises auf die Patenterteilung: **01.12.2010**
(21) Anmeldenummer: 05700842.7
(22) Anmeldetag: 12.01.2005
(51) Int. Cl.: A61K 31/728, A61P 17/00, A61P 27/02

(54) **MITTEL ZUR BEHANDLUNG VON ENTZÜNDLICHEN ERKRANKUNGEN**
AGENT FOR TREATING INFLAMMATORY DISEASES
AGENT SERVANT A TRAITER DES MALADIES INFLAMMATOIRES

(30) Priorität: 14.01.2004 DE 102004002001
(43) Veröffentlichungstag der Anmeldung: 18.10.2006
(73) Patentinhaber: Reinmüller, Johannes, 65193 Wiesbaden (DE); Dirting, Kay, 65189 Wiesbaden (DE)
(72) Erfinder: Reinmüller, Johannes, 65193 Wiesbaden (DE); Dirting, Kay, 65189 Wiesbaden (DE)
(74) Vertreter: Weiss, Wolfgang
(86) Internationale Anmeldenummer: PCT/EP2005/000215
(87) Internationale Veröffentlichungsnummer: WO 2005/067944

(56) Entgegenhaltungen:
- EP-A- 0 715 852
- WO-A-00/44367
- DE-A1- 10 209 966
- US-A- 4 141 973
- US-A- 5 679 655
- US-A- 5 830 882
- US-A- 5 910 489
- "Artificial skin powder composition containing sodium hyaluronate and its preparation" DERWENT, 25. Juli 2000 (2000-07-25), XP002232150

## Beschreibung

Die vorliegende Erfindung betrifft die Verwendung von Hyaluronsäure zur Behandlung entzündlicher Erkrankungen, insbesondere Haut- oder Schleimhauterkrankungen.

Eine große Anzahl von Hauterkrankungen, insbesondere die des atopischen Formenkreises, sind ursächlich nicht erklärt. Gemeinsam sind diesen Erkrankungen Entzündungsreaktionen der Lederhaut (Dermis) und der dermo-epithelialen Übergangszone. Es ist bekannt, dass bei diesen Erkrankungen erhebliche Verschiebungen des normalen Hyaluronsäuregehaltes in Dermis und Epidermis auftreten. Die Behandlung solcher Erkrankungen besteht derzeit in verschiedenen Maßnahmen, z.B. Verabreichung von fetthaltigen Salben, Cremes oder Lotionen mit unterschiedlichem Wirkstoffzusatz. Am effektivsten werden solche entzündlichen Erkrankungen jedoch mit corticoidhaltigen Zubereitungen zur äußerlichen (topischen) Anwendung behandelt. Dabei werden die hinreichend bekannten lokalen und systemischen Nebenwirkungen der Corticoide (Abkömmlinge des körpereigenen Steroidhormons Cortisol) in Kauf genommen. Bei chronischer Anwendung der topischen Corticoidzubereitungen kommt es in der Regel zu Spätfolgen, wie z.B. Hautatrophie.

Ein anderer Behandlungsweg besteht in der Anwendung von Mitteln, die an Zellen des Immunsystems angreifen und die Biosynthese von Immunmodulatoren hemmen, wie z.B. Cyclosporin, Tacrolismus und Pimecrolismus. Substanzen mit derartigen Wirkungen werden auch als Immunsuppressiva bezeichnet, weil sie die Immunantwort eines Bioorganismus unterdrücken. Damit ist ihre Anwendung erheblich eingeschränkt, da ein intaktes Immunsystem wesentlich für einen dauerhaft zufriedenstellenden Gesundheitszustand ist. Die Anwendung kommt somit nur bei schweren Krankheitserscheinungen und bei körperlich ausgereiften Individuen infrage. Völlig ungeklärt sind Langzeitrisiken und die Risiken einer Langzeitanwendung, wie z.B. Cancerogenität.

Es besteht daher ein Bedürfnis, neue Mittel zur Behandlung von entzündlichen Haut- oder Schleimhauterkrankungen zu entwickeln, bei denen die Nachteile des Standes der Technik mindestens teilweise vermieden werden können.

Aus dem Stand der Technik (DE10209966) ist die Anwendung von pharmazeutischen Zusammensetzungen, die vernetzte oder unvernetzte und vorzugsweise langkettige Hyaluronsäure sowie übliche pharmazeutische Hilfs- oder/und Trägerstoffe enthalten bekannt. Allerdings beschreibt dieser Stand der Technik nicht die Verwendung der Hyaluronsäure enthaltenden Zusammensetzung zur Behandlung entzündlicher Hauterkrankungen.

Überraschenderweise wurde gefunden, dass Hyaluronsäure, ein Glykosaminoglykan, sich für die Behandlung entzündlicher Haut- oder Schleimhauterkrankungen, insbesondere von entzündlichen Hauterkrankungen des atopischen Formenkreises, hervorragend eignet.

Ein Gegenstand der Erfindung ist somit die Verwendung von Hyaluronsäure in vernetzter Form zur Herstellung eines Mittels für die Vorbeugung oder Behandlung entzündlicher Hauterkrankungen, ausgewählt aus atopischer Dermatitis, Ekzemen wie seborrhoischen und mikrobiellen Ekzemen, Pruritus, Prurigo, Urtikaria, Lichen ruber, Psoriasis wie Psoriasis vulgaris, Vitiligo, viralen Hauterkrankungen, die zur Warzenbildung führen wie Verruca vulgaris oder Condylomata accuminata, Rosacea, perioraler Dermatitis, Akne wie Akne vulgaris oder Akne conglobata.

Ein weiterer Gegenstand der Erfindung ist ein Verfahren zur Vorbeugung oder Behandlung einer entzündlichen Hauterkrankung, wobei man einem zu behandelnden Subjekt, beispielsweise einem menschlichen Patienten oder auch einem Tier, ein Präparat verabreicht, das vernetzte Hyaluronsäure in einer zur Behandlung der Erkrankung ausreichenden Menge enthält.

Die Verabreichung von Hyaluronsäure kann grundsätzlich auf beliebige Art und Weise erfolgen, sofern diese zur Behandlung der jeweiligen Erkrankung geeignet ist. In vielen Fällen erfolgt eine lokale Verabreichung im Bereich der erkrankten Hautstelle, z.B. einer Läsion. Bevorzugt erfolgt die Verabreichung intradermal, z.B. durch Injektion, oder durch topische Anwendung auf die erkrankte Hautstelle.

Zur Behandlung entzündlicher Hauterkrankungen ist Hyaluronsäure sowohl in unvernetzter als auch in vernetzter Form oder Mischungen davon geeignet. Unvemetzte Hyaluronsäure wird vorzugsweise ausgewählt aus (i) langkettiger Hyaluronsäure mit einen durchschnittlichen Molekulargewicht (Gewichtsmittel) von mindestens 200 kD und (ii) kurzkettiger Hyaluronsäure mit einen durchschnittlichen Molekulargewicht (Gewichtsmittel) bis zu 50 kD oder Mischungen davon.

Vernetzte Hyaluronsäure kann z.B. kovalent oder nicht-kovalent vernetzt sein. Die Herstellung der vernetzten Hyaluronsäure kann an sich auf bekannte Weise erfolgen. Die kovalente Vernetzung erfolgt dabei im Allgemeinen durch Vernetzung mit bifunktionellen reaktiven Agentien, wie z.B. Glutaraldehyd oder Carbodiimid, über bifunktionelle Aminosäuren, z.B. Lysin, Protamine oder Albumine. Es können z.B. aber auch Vernetzungen über eine Amid-, Ester- oder Etherbindung hergestellt werden. Weitere geeignete Reagenzien zur kovalenten Vernetzung von Hyaluronsäure sind Ethylenglykol- oder 1,4-Butandiol-diglycidether, Divinylsulfon, Photoquervemetzungsreagenzien, wie Ethyleosin, Hydrazide, wie Bishydrazid-, Trishydrazid- und polyvalente Hydrazidverbindungen. Weiterhin können auch intra- oder/und intermolekular veresterte Hyaluronsäurederivate eingesetzt werden. Besonders bevorzugt ist eine nicht-kovalente Quervernetzung unter Verwendung mehrwertiger Metallionen, wie etwa Eisen, Kupfer, Zink, Calcium, Magnesium, Barium und anderen chelatierenden Metallionen.

Hyaluronsäure ist im vernetzten Zustand handelsüblich erhältlich (z.B. Hylaform^{®}, eine vernetzte Hyaluronsäure der Fa. Biomatrix, NJ, USA; zur Herstellung vgl. auch US-A-4 713 448, US-A-4 605 691, APC^{®} der Fa. Fidia, Incert^{®} der Fa. Anika Therapeutics, Intergel^{®} der Fa. LifeCore oder Restylane^{®} der Fa. Q-Med).

Bei der Anwendung ist von Bedeutung das Molekulargewicht sowie bei vernetzten Hyaluronsäureprodukten der Vernetzungsgrad, der beispielsweise im Bereich von 0,1 % bis 10 % liegt, ohne darauf begrenzt zu sein. Generell ist festzustellen, dass bei langkettiger Hyaluronsäure ein geringerer Vernetzungsgrad ausreicht, um eine gelartige Matrix zu erhalten, während bei kurzkettiger Hyaluronsäure ein höherer Vernetzungsgrad erforderlich ist, um vergleichbare Eigenschaften zu erhalten.

Das Hyaluronsäurepräparat kann sowohl in der Humanmedizin als auch in der Veterinärmedizin, beispielsweise zur Behandlung von Haustieren oder Nutztieren eingesetzt werden.

Die pharmazeutischen Zusammensetzungen enthalten die Hyaluronsäure vorzugsweise in Mengen von 0,01 bis 20 Gew.-%, bezogen auf die gesamte pharmazeutische Zusammensetzung, insbesondere in einer Menge von 0,01 bis 5 Gew.-% und besonders bevorzugt in einer Menge von 0,01 bis 1 Gew.-%.

Als pharmazeutische Hilfsstoffe können die pharmazeutischen Zusammensetzungen z.B. Mittel zur pH-Wert-Einstellung, Stabilisierungsmittel, Antioxidantien, Lösungsvermittler, penetrationsfördernde Mittel, Konservierungsmittel oder/und Gelbildner enthalten, wie sie in derartigen Zusammensetzungen üblicherweise verwendet werden, Sie werden in den in derartigen Zubereitungen üblichen Mengen verwendet.

Die pharmazeutischen Zusammensetzungen können gegebenenfalls neben dem Wirkstoff Hyaluronsäure auch noch weitere pharmazeutische Wirkstoffe, die mit der Hyaluronsäure im Rahmen der Applikation verträglich sind, enthalten, z.B. Wirkstoffe zur Therapie von Hauterkrankungen (Dermatosen), Antimykotika, Antibiotika (z.B. Gentamycin, Vancomycin, Penicilline oder Cephalosporine), Sulfonamide, Desinfektionsmittel, Hormone (z.B. Corticoide) und Hormonabkömmlinge (z.B. Cortisol), lokale Anästhetika (z.B. vom Typ des Lidocains oder Novocains), vasoaktive Substanzen zur Gefäßkonstriktion (Vermeidung von Blutungen), Adrenalin, Enzyme (wie z.B. Hyaluronidase), Interleukine, Wachstumsfaktoren (z.B. EGF, PDGF oder/und IGF), Vitamine (z.B. Vitamin D), Hautpflegemittel und/oder durchblutungsfördernde (hyperämisierende) Mittel. Die weiteren Wirkstoffe können gegebenenfalls mit der Hyaluronsäure assoziiert sein, z.B. durch kovalente oder nicht-kovalente Wechselwirkungen.

Von Bedeutung sind auch Zusatzstoffe, wie z.B. zwei- oder dreiwertige Metallionen, die durch Chelatbildung quervernetzend und stabilisierend wirken können, die andererseits auch den Abbau der wirksamen Hyaluronsäure beschleunigen können.

Im Gewebe erfolgt der Abbau von Hyaluronsäure natürlicherweise durch eine Vielzahl unterschiedlicher Hyaluronidasen oder durch Sauerstoffradikale. Daher sind weiterhin von Bedeutung Zusatzstoffe, die hemmend auf Hyaluronidasen einwirken (Heparin, Indomethazin oder/und Salizylate) und solche, die den oxidativen Abbau der Hyaluronsäure im Gewebe als so genannte Radikalfänger verhindern (Vitamine A, E oder/und C).

Eine besonders bevorzugte Ausführungsform der Zubereitung sind Mischungen aus langkettiger Hyaluronsäure (≥ 200 kD) mit kurzkettiger Hyaluronsäure (z-B. Hexamere der repetitiven Disaccharideinheiten oder größere Einheiten bis 50 kD) oder auch Mischungen der vorgenannten mit quervernetzter Hyaluronsäure. Dabei entstehen visköse injizierbare Zubereitungen, die bevorzugt mit Injektionskanülen (z.B. 30 gauge) intradermal oder an die Grenze des dermo-epidermalen Übergangs durch Injektion flächenhaft eingebracht werden. Auch das Setzen einzelner Quaddeln ist erfindungsgemäß geeignet. Den injizierbaren Hyaluronsäure-Zubereitungen können zur Minimierung der Schmerzhaftigkeit des Einstichs die bekannten Lokalanästhetika zugesetzt werden.

Eine weitere besonders bevorzugte Ausführungsform sind Mischungen aus quervernetzter Hyaluronsäure und nicht quervernetzter Hyaluronsäure.

Anstelle der Injektionstechnik mit Kanülen lassen sich die Zubereitungen äußerst wirksam auch durch Druckinjektion anwenden. Dieses Verfahren zeichnet sich durch weitgehende Schmerzfreiheit aus.

Weitere Zubereitungen des Wirkstoffs sind wässrige Lösungen oder Emulsionen zur intravenösen Applikation oder zur Instillation in Körperhöhlen oder Hohlorgane.

Da durch den Entzündungsprozess der gesamten Haut im Falle einer der vorgenannten Erkrankungen die Barrierefunktion des dermo-epithelialen Übergangs und der tieferen Epidermisschichten gestört ist, können die Zubereitungen auch topisch, d.h. oberflächlich auf die Haut in Form von Salben, Cremes, Lotionen, Gelen, Sprays, Tinkturen, Shampoos, Okklusivfolien aufgebracht werden. Eine besondere Zubereitungsform im Sinne der Erfindung ist eine trockene Hyaluronsäure-Zubereitung in Form eines Puders, welches insbesondere zur Behandlung nässender Ekzeme Verwendung findet. Auch das Einbringen der Wirkstoffe in mikroverkapselter Form bzw. in Form von Liposomen ist Gegenstand der Erfindung. Eine Vielzahl solcher topischer Zubereitungen sind bekannt aus dem Bereich der Kosmetik, wobei diese Substanzen ausschließlich auf gesunde und intakte Haut aufgebracht werden.

Überraschend hat sich herausgestellt, dass bei erfindungsgemäßer Anwendung solcher topischer Zubereitungen auf entzündliche Haut nicht etwa eine Exazerbation sondern eine Rückbildung der entzündlichen Erscheinungen eintritt. Soweit Schleimhäute der Atemwege behandelt werden müssen, können auch Aerosole als Inhalaltionslösungen eingesetzt werden.

Die Herstellung der Zusammensetzungen kann auf eine für die Herstellung derartiger Zusammensetzungen an sich übliche, allgemein bekannte Weise erfolgen. Dabei ist die Reihenfolge der Vermischung der einzelnen Bestandteile in der Regel nicht kritisch.

Die Art, Dosis und Häufigkeit der Verabreichung der Zusammensetzung sowie die Beschaffenheit (z.B. Viskosität, Vernetzungsgrad, Wirkstoffgehalt etc.) richten sich insbesondere nach der Art und Schwere der Erkrankung sowie nach dem Alter des Patienten und dem Ort und der Art der Applikation, z.B. dem Zustand und der Empfindlichkeit der entzündeten Stelle. Werden die Zusammensetzungen in Form topisch applizierbarer Zubereitungen verabreicht, so entspricht die Verabreichung in der Regel den für derartige Zusammensetzungen üblichen Bedingungen.

Die Art der Behandlung und die Häufigkeit der Applikation richtet sich insbesondere auch nach dem individuellen Ansprechen der zu behandelnden Personen. Vorzugsweise erfolgt eine Applikation von Gelen oder Lösungen in Abständen von mehreren Tagen bis zu ein oder zwei Monaten, insbesondere ca. ein bis zwei Wochen.

Die Erfindung beinhaltet auch Mischungen der Hyaluronsäure mit anderen Glykosaminoglykanen in vernetzter oder/und nicht vernetzter Form. Zu möglichen Kombinationen und Quervernetzungsmöglichkeiten wird Bezug genommen auf EP-B-0 619 737, DE-A-102 99 66 und WO 03/041723. Mischungen aus Hyaluronsäure und Heparin sind bevorzugt. Weiterhin bevorzugt sind Mischungen aus Hyaluronsäure und positiv geladenen Glykosaminoglykanen, wie Chitosamin.

Besonders erfolgreich können mit den Zubereitungen folgende Hauterkrankungen behandelt werden: atopische Dermatitis bzw. ekzematöse Hauterkrankungen, wie seborrhoisches Ekzem und mikrobielles Ekzem, Pruritus, Prurigo, Urtikaria, Lichen ruber, Psoriasis, wie Psoriasis vulgaris, Vitiligo, Rosacea, periorale Dermatitis, Akne vulgaris oder Akne conglobata sowie chronische und akute Ulcerationen der Haut. Des Weiteren ist die Zusammensetzung zur unterstützenden Behandlung bei Mykosen, insbesondere in Kombination mit einem Antimykotikum geeignet.

Überraschenderweise lassen sich mit den Zubereitungen auch virale Hauterkrankungen, die zu Warzenbildung führen, günstig beeinflussen, wie z.B. Verruca vulgaris, Condylome, wie z.B. Condylomata accuminata, oder andere durch Viren der Papillomgruppe ausgelösten krankhaften Hauterscheinungen. Hyaluronsäure wird dabei bevorzugt an die Basis solcher Läsionen am dermo-epidermalen Übergang injiziert. Bekannt war die Wirkung von Hyaluronsäure auf Herpesviren (siehe Patentanmeldung WO 03/041723).

Eine weitere Erkrankungsgruppe der Schleimhäute sind so genannte Aphten. Dabei bilden sich schmerzhafte Bläschen auf den Schleimhäuten. Die Ursache für Aphtenbildung ist bisher nicht bekannt. Die Anwendung von Hyaluronsäure bei Aphten führt zu sofortiger Rückbildung der Schmerzhaftigkeit und zu einer nachfolgenden Abheilung der Aphten.

Auch die Anwendungen der Hyaluronsäure zur Vorbeugung oder Behandlung von Erkrankungen von Schleimhäuten oder von Körperöffnungen, die mit solchen ausgekleidet sind, werden offenbart. Hierbei können geeignete gelartige Zubereitungen in entsprechende Hohlräume instilliert werden oder die Schleimhautläsionen können durch geeignete Techniken submucös unterlegt werden. Behandlungserfolge sind z.B. zu erreichen bei polypösen Schleimhauterkrankungen, wie Nasenpolyposis, oder bei entzündlichen Darmerkrankungen. Die submucöse Verabreichung des Wirkstoffs kann in diesem Fall auch endoskopisch erfolgen.

Die Anwendung der Zubereitung erstreckt sich nicht nur auf das Einbringen in Dermis und Epidermis. In besonderen Fällen kann auch die Applikation der Zubereitung unter die Dermis erforderlich sein und ist somit eine Anwendung im Sinne der Erfindung. Auch das Einbringen des Wirkstoffs in abgeschlossene Körperhöhlen, wie z.B. Brust- und Bauchhöhle, ist erfindungsgemäß.

Weiterhin offenbart ist die Verwendung einer Zubereitung, die eine Mischung aus vernetzter und nicht vernetzter Hyaluronsäure enthält, für kosmetische oder pharmazeutische Anwendungen, insbesondere zur Behandlung von Haut- oder Weichteildefekten, sowie Falten der Haut.

Von der Anwendung vernetzter Hyaluronsäure zur Unterlegung von Hautfalten und ähnlichen Defekten sind als Nebenwirkungen entzündliche Reaktionen bekannt, die sich in Form von Rötungen, Schwellungen, Brennen, Jucken und mit Ausbildung kleiner intradermaler Knötchen darstellen. Diese Nebenwirkungen lassen sich unterbinden, indem den Zubereitungen aus ausschließlich vernetzter Hyaluronsäure nicht vernetzte Hyaluronsäure beigemischt wird. Als günstig haben sich dabei Beimischungen aus niedermolekularen Fraktionen bis ca. 500 kD erwiesen. Der protektive Effekt kann aber auch mit nicht vernetzter Hyaluronsäure oberhalb der genannten Molekülgröße erzielt werden, wobei auch Fraktionen bis 5 Mio D eingesetzt werden können. Daher sind Mischungen aus vernetzter und nicht vernetzter Hyaluronsäure zum Einsatz in der kosmetischen oder pharmazeutischen Behandlung von Falten, Weichteildefekten und zur augmentativen Behandlung von Weichteilen (z.B. Lippen) ebenfalls offenbart. Bezüglich weiterer bevorzugter Ausgestaltungen für diesen Gegenstand wird auf die Ausführungen oben verwiesen.

Zur Anwendung zählt auch der Einsatz der Hyaluronsäure an der Bindehaut des Auges und an der Hornhaut, wobei bisher lediglich die Anwendung nicht vernetzter Hyaluronsäure nach Laser-Ablation der Hornhaut bekannt ist. Es hat sich gezeigt, dass auch die vernetzte Hyaluronsäure und Mischungen aus vernetzter und nicht vernetzter Hyaluronsäure bei Manipulationen, Verletzungen und Entzündungen der Hornhaut eingesetzt werden können. Auch bei formenden Behandlungen an der Hornhaut des Auges wird der Einsatz der Hyaluronsäure zwischen Hornhaut und formender Schale im Sinne einer Verbesserung der Sehkraft beschrieben. Bei der Einpflanzung von Kunststofflinsen in das Auge zur Beseitigung des grauen Stars (Katarakt) wird zur Zeit nicht vernetzte Hyaluronsäure zur Lubrikation auf die Oberfläche der Kunststofflinse aufgebracht. Das Aufbringen der Zubereitung, insbesondere einer Mischung aus nicht vernetzter und vernetzter Hyaluronsäure, gegebenenfalls in Kombination mit einem weiteren Glykosaminoglykan, wie Heparin, ist dadurch vorteilhafter, indem die postoperative Entzündungsreaktion langfristig unterdrückt wird und damit dem Auftreten des so genannten Nachstars vorgebeugt wird. An der Bindehaut des Auges werden alle entzündlichen Veränderungen, also Bindehautentzündung bzw. Conjunctivitis, günstig beeinflusst. Bezüglich weiterer Ausgestaltungen für diesen Gegenstand wird auf die Ausführungen oben verwiesen.

### Anwendungsbeispiele

### Beispiel 1

Eine 30jährige Patientin mit atopischer Dermatitis wurde erfindungsgemäß behandelt, indem 0,1 ml einer langkettigen Hyaluronsäure (Hyal System, Fa. Merz, Frankfurt/M) intradermal unter atopische Läsionen im Bereich der Ellenbeuge flächenhaft durch Injektion eingebracht wurde. Bereits 3 Tage nach der Behandlung war der zuvor quälende Juckreiz abgeklungen. Die ursprünglichen entzündlichen Erscheinungen waren deutlich rückläufig.

### Beispiel 2

Bei einem 14jährigen wurde eine Viruswarze am Zelgefinger rechts mit 0,1 ml quervernetzter Hyaluronsäure (Juvederm 18, Fa. LEA Derm, Hallbergmoos) intraläsional infiltriert. Nach 4 Wochen bestand in der behandelten Region intakte Haut ohne Warzenbefall.

### Beispiel 3

Einem 20jährigen Mann mit Akne vulgaris im Gesicht wurden die befallenen Hautareale mit Hylaform 0,75 ml tief intradermal injiziert. Nach 4 Wochen waren die akuten entzündlichen Veränderungen der Haut abgeklungen- Ein erneutes Auftreten der Erkrankung konnte für 6 Monate unterdrückt werden.

## Patentansprüche

1. Verwendung von Hyaluronsäure in vernetzter Form zur Herstellung eines Mittels zur Vorbeugung oder Behandlung entzündlicher Hauterkrankungen, ausgewählt aus atopischer Dermatitis, Ekzemen wie seborrhoischen und mikrobiellen Ekzemen, Pruritus, Prurigo, Urtikaria, Lichen ruber, Psoriasis wie Psoriasis vulgaris, Vitiligo, viralen Hauterkrankungen, die zur Warzenbildung führen wie Verruca vulgaris oder Condylomata accuminata, Rosacea, perioraler Dermatitis, Akne wie Akne vulgaris oder Akne conglobata.

2. Verwendung nach Anspruch 1,
**dadurch gekennzeichnet,**
**dass** ein Mittel zur lokalen Verabreichung hergestellt wird.

3. Verwendung nach Anspruch 1 oder 2,
**dadurch gekennzeichnet,**
**dass** ein Mittel zur intradermalen Verabreichung, zur Verabreichung an der Grenze des dermo-epithelialen Übergangs oder zur topischen Verabreichung hergestellt wird.

4. Verwendung nach einem der Ansprüche 1 bis 3,
**dadurch gekennzeichnet,**
**dass** der Vernetzungsgrad im Bereich von 0,1 % bis 10 % liegt.

5. Verwendung nach einem der Ansprüche 1 bis 4,
**dadurch gekennzeichnet,**
**dass** die vernetzte Hyaluronsäure im Gemisch mit unvernetzter Hyaluronsäure vorliegt.

6. Verwendung nach Anspruch 5,
**dadurch gekennzeichnet,**
**dass** die unvernetzte Hyaluronsäure aus
(i) langkettiger Hyaluronsäure mit einen durchschnittlichen Molekulargewicht (Gewichtsmittel) von mindestens 200 kD und
(ii) kurzkettiger Hyaluronsäure mit einen durchschnittlichen Molekulargewicht (Gewichtsmittel) bis zu 50 kD oder Mischungen davon ausgewählt wird.

7. Verwendung nach einem der Ansprüche 1 bis 6,
**dadurch gekennzeichnet,**
**dass** das Mittel weiterhin einen Hemmstoff des Hyaluronsäureabbaus enthält.

8. Verwendung nach Anspruch 7,
**dadurch gekennzeichnet,**
**dass** der Hemmstoff des Hyaluronsäureabbaus ausgewählt wird aus Heparin, Indomethazin, Salicylaten, Radikalfängern, wie Vitamin A, C oder E, und Mischungen davon.

9. Verwendung nach einem der Ansprüche 1 bis 8,
**dadurch gekennzeichnet,**
**dass** die Hyaluronsäure als injizierbare Zubereitung, Salbe, Creme, Lotion, Gel, Spray, Tinktur, Shampoo, Okklusivfolie, Puder oder inhalierbare Zubereitung vorliegt.

10. Verwendung nach einem der Ansprüche 1 bis 9,
**dadurch gekennzeichnet,**
**dass** das Mittel weiterhin ein anderes Glykosaminoglykan in vernetzter oder unvernetzter Form enthält.

11. Verwendung nach einem der Ansprüche 1 bis 10 in der Humanmedizin.

12. Verwendung nach einem der Ansprüche 1 bis 11 in der Veterinärmedizin.

## Claims

1. Use of hyaluronic acid in crosslinked form for the production of a composition for preventing or treating inflammatory skin diseases, selected from atopic dermatitis, eczemas, such as seborrheic and microbial eczemas, pruritus, prurigo, urticaria, red lichen, psoriasis, such as common psoriasis, vitiligo, viral skin diseases which lead to wart formation, such as common verruca or vulvar warts, rosacea, perioral dermatitis, acne, such as common acne or acne conglobata.

2. Use according to Claim 1,
**characterized in that**
a composition for local administration is produced.

3. Use according to Claim 1 or 2,
**characterized in that**
a composition for intradermal administration, for administration to the border of the dermoepithelial transition or for topical administration is produced.

4. Use according to one of Claims 1 to 3, **characterized in that**
the degree of crosslinking is in the range from 0.1% to 10%.

5. Use according to one of Claims 1 to 4, **characterized in that**
the crosslinked hyaluronic acid is present in a mixture with uncrosslinked hyaluronic acid.

6. Use according to Claim 5,
**characterized in that**
the uncrosslinked hyaluronic acid is selected from
(i) long-chain hyaluronic acid having an average molecular weight (weight-average) of at least 200 kD
and
(ii) short-chain hyaluronic acid having an average molecular weight (weight-average) up to 50 kD or mixtures thereof.

7. Use according to one of Claims 1 to 6, **characterized in that**
the composition furthermore contains an inhibitor of hyaluronic acid degradation.

8. Use according to Claim 7,
**characterized in that**
the inhibitor of hyaluronic acid degradation is selected from heparin, indomethacin, salicylates, free radical traps, such as vitamin A, C or E, and mixtures thereof.

9. Use according to one of Claims 1 to 8, **characterized in that**
the hyaluronic acid is present as an injectable preparation, ointment, cream, lotion, gel, spray, tincture, shampoo, occlusive film, powder or inhalable preparation.

10. Use according to one of Claims 1 to 9, **characterized in that**
the composition furthermore contains another glycosaminoglycan in crosslinked or uncrosslinked form.

11. Use according to one of Claims 1 to 10 in human medicine.

12. Use according to one of Claims 1 to 11 in veterinary medicine.

## Revendications

1. Utilisation de l'acide hyaluronique sous forme réticulée pour la production d'un agent pour la prévention ou le traitement des maladies cutanées inflammatoires, choisies parmi la dermatite atopique, les eczémas comme les eczémas séborrhéiques et microbiens, le prurit, le prurigo, l'urticaire, le lichen ruber, le psoriasis comme le psoriasis vulgaire, le vitiligo, les maladies cutanées virales qui conduisent à la formation de verrues comme la verrue vulgaire ou le condylome acuminé, la rosacée, la dermatite périorale, l'acné comme l'acné vulgaire ou l'acné conglobata.

2. Utilisation selon la revendication 1 **caractérisée en ce qu'**un agent pour l'administration locale est produit.

3. Utilisation selon la revendication 1 ou 2 **caractérisée en ce qu'**un agent pour l'administration intradermique, pour l'administration à la limite de la jonction dermo-épithéliale ou pour l'administration topique est produit.

4. Utilisation selon l'une des revendications 1 à 3 **caractérisée en ce que** le degré de réticulation est situé dans le domaine de 0,1 % à 10 %.

5. Utilisation selon l'une des revendications 1 à 4 **caractérisée en ce que** l'acide hyaluronique réticulé est présent en mélange avec de l'acide hyaluronique non réticulé.

6. Utilisation selon la revendication 5 **caractérisée en ce que** l'acide hyaluronique non réticulé est choisi parmi
(i) un acide hyaluronique à longue chaîne ayant une masse moléculaire moyenne (moyenne en poids) d'au moins 200 kD et
(ii) un acide hyaluronique à courte chaîne ayant une masse moléculaire moyenne (moyenne en poids) de jusqu'à 50 kD ou leurs mélanges.

7. Utilisation selon l'une des revendications 1 à 6 **caractérisée en ce que** l'agent contient en outre un inhibiteur de la dégradation de l'acide hyaluronique.

8. Utilisation selon la revendication 7 **caractérisée en ce que** l'inhibiteur de la dégradation de l'acide hyaluronique est choisi parmi l'héparine, l'indométhazine, les salicylates, les piégeurs de radicaux, comme la vitamine A, C ou E, et leurs mélanges.

9. Utilisation selon l'une des revendications 1 à 8 **caractérisée en ce**
**que** l'acide hyaluronique est présent sous forme de préparation injectable, de pommade, de crème, de lotion, de gel, de spray, de teinture, de shampoing, de feuille occlusive, de poudre ou de préparation inhalable.

10. Utilisation selon l'une des revendications 1 à 9 **caractérisée en ce que** l'agent contient en outre un autre glycosaminoglycane sous forme réticulée ou non réticulée.

11. Utilisation selon l'une des revendications 1 à 10 en médecine humaine.

12. Utilisation selon l'une des revendications 1 à 11 en médecine vétérinaire.
